(19) Europäisches Patentamt
European Patent Office
Office européen des brevets

(11) **EP 3 335 809 B1**

(12) **EUROPEAN PATENT SPECIFICATION**

(45) Date of publication and mention of the grant of the patent:
**27.05.2020 Bulletin 2020/22**

(21) Application number: **15900981.0**

(22) Date of filing: **10.08.2015**

(51) Int Cl.:
*B09B 3/00* *(2006.01)*     *A61L 2/04* *(2006.01)*
*A61L 11/00* *(2006.01)*    *C01B 3/22* *(2006.01)*
*C01B 3/38* *(2006.01)*     *C12P 7/06* *(2006.01)*
*B09B 5/00* *(2006.01)*

(86) International application number:
**PCT/JP2015/072628**

(87) International publication number:
**WO 2017/026027 (16.02.2017 Gazette 2017/07)**

(54) **METHOD FOR EFFECTIVELY UTILIZING ENERGY IN WASTE-INCINERATION FACILITY WITH ETHANOL PRODUCTION EQUIPMENT**

VERFAHREN ZUR EFFEKTIVEN NUTZUNG VON ENERGIE IN EINER MÜLLVERBRENNUNGSANLAGE MIT ETHANOLHERSTELLUNGSAUSRÜSTUNG

PROCÉDÉ PERMETTANT D'UTILISER EFFICACEMENT L'ÉNERGIE DANS UNE INSTALLATION D'INCINÉRATION DE DÉCHETS AVEC UN ÉQUIPEMENT DE PRODUCTION D'ÉTHANOL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**20.06.2018 Bulletin 2018/25**

(73) Proprietor: **Hitachi Zosen Corporation**
**Osaka-shi, Osaka 559-8559 (JP)**

(72) Inventors:
• **SERA, Yutaka**
  **Osaka-shi**
  **Osaka 559-8559 (JP)**
• **TOMIYAMA, Shigeo**
  **Osaka-shi**
  **Osaka 559-8559 (JP)**
• **NAKAMORI, Ken-ichi**
  **Osaka-shi**
  **Osaka 559-8559 (JP)**

(74) Representative: **Grünecker Patent- und Rechtsanwälte**
**PartG mbB**
**Leopoldstraße 4**
**80802 München (DE)**

(56) References cited:
| | |
|---|---|
| EP-A1- 1 829 821 | WO-A1-2007/005954 |
| WO-A1-2010/146243 | JP-A- S5 326 768 |
| JP-A- S5 333 867 | JP-A- 2005 211 805 |
| JP-A- 2006 325 577 | JP-A- 2008 104 452 |
| JP-A- 2008 104 452 | US-A1- 2010 105 127 |

**Description**

Technical Field

The present invention relates to a method for effectively utilizing energy generated in a waste-incineration facility annexed with an ethanol production equipment that produces ethanol by enzymatically saccharifying cellulose biomass containing mainly papers contained in municipal solid wastes i.e.,"MSW" to monosaccharides as the constituent sugars, and further fermenting the monosaccharides.

Background Art

The applicant has proposed a method for effectively utilizing energy in a waste-incineration facility annexed with an ethanol production equipment (PTL 1). In this method, municipal solid wastes collected in the form of a mixture of various kinds of wastes are roughly shredded; the resulting roughly shredded matter is separated into light components having a small specific gravity (mainly containing papers and plastics) and heavy components containing a large amount of water and having a large specific gravity (mainly containing kitchen wastes, i.e., garbage) by a shredding sorter; the light components are pulped with water added thereto to defiber paper, from which plastics (which are not defibered) are separated; and ethanol is produced from the resulting pulp as a raw material slurry.

A boiler using waste heat generated from the waste-incineration facility and a steam turbine using steam generated in the boiler are annexed to the incineration facility, and the incineration waste heat, and the boiler steam generated from the boiler and/or the extracted steam generated from the steam turbine are utilized as a heat source for the yeast cultivation (at approximately 30°C), the saccharification and fermentation (at approximately 40°C), and the distillation (at approximately 80°C) in the ethanol production process, thereby decreasing the ethanol production cost and the $CO_2$ emission.

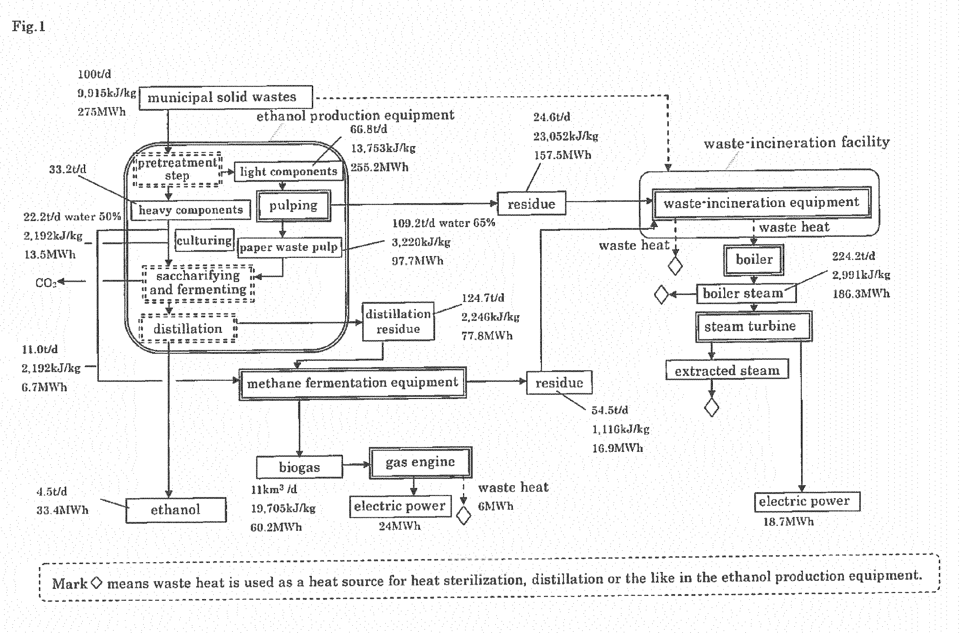

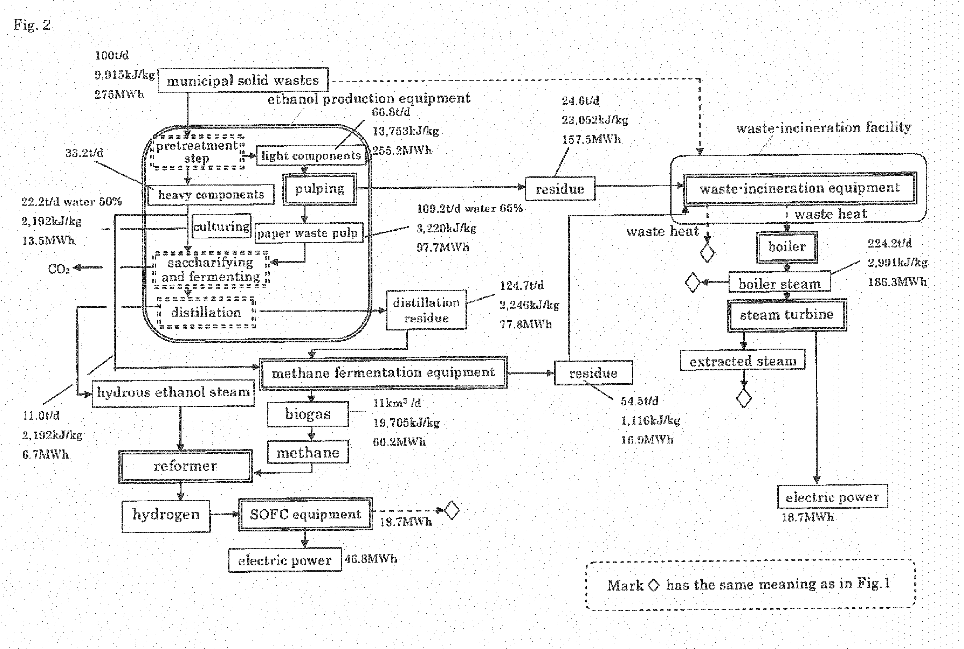

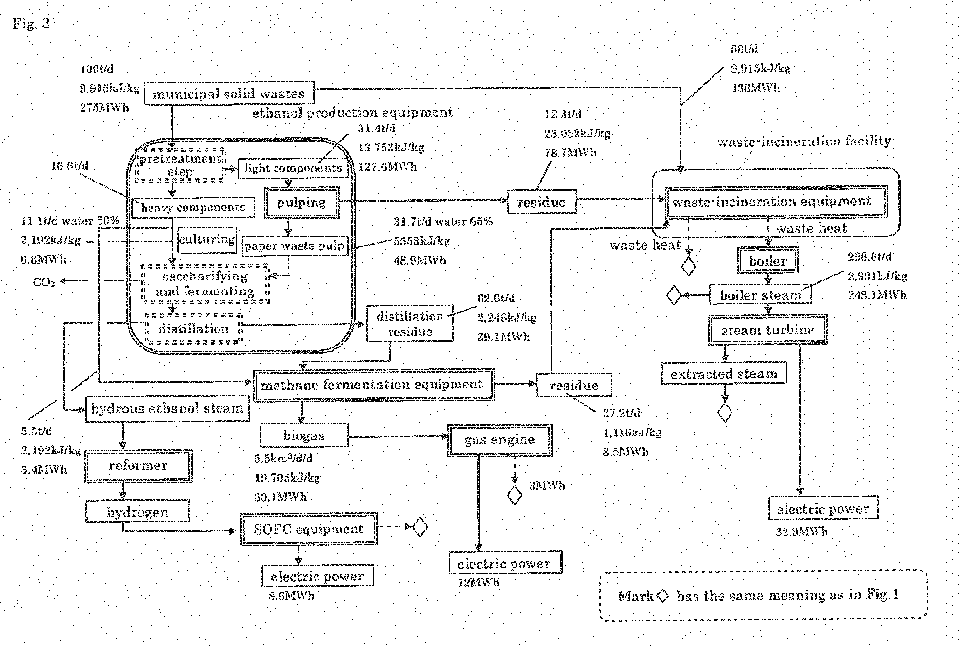

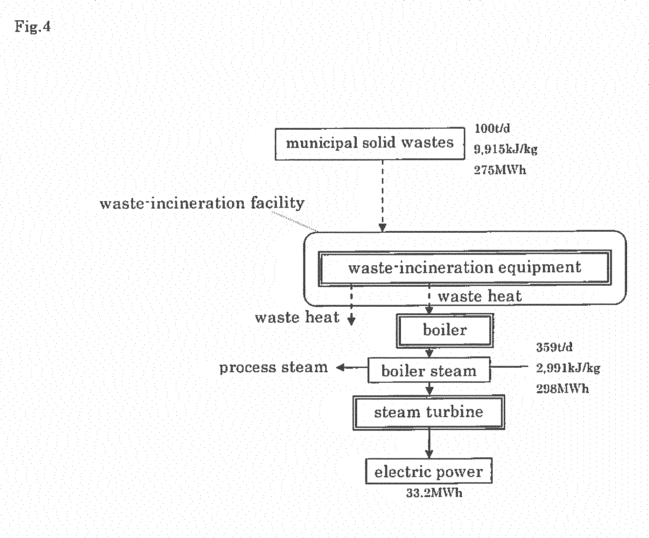

The techniques of utilizing waste heat from a waste-incineration facility and a fuel cell as a heat source for a production process of a biogas or bioethanol have been known (see PTLs 2 and 3, NPL 1, and the like). For example, PTL 2 discloses in paragraphs [0026] to [0027] that waste heat generated from a waste-incineration facility is effectively utilized by feeding to a dry methane fermentation facility, and the utilization of waste heat is disclosed in Figs. 3 and 4. PTL 3 discloses in paragraph [0049] that waste heat generated from a fuel cell is utilized as a heat source for distillation separation of alcohol components in a separation and recovery section, and utilized for keeping the heat of the fermentation section, and the utilization of waste heat is disclosed in Fig. 2. NPL 1 is the academic journal publishing the article entitled "Production of hydrogen for fuel cells by reformation of biomass-derived ethanol", and discloses in Fig. 1 in page 146 that waste heat generated from a fuel cell and others is effectively utilized by feeding to an anaerobic digestion process and the like.

WO2007/005954A1 discloses an integrated thermochemical and biocatalytic energy production apparatus and method using organic waste. Document JP 2005 211 805 A discloses a method for producing ethanol in waste incineration facilities.

Citation List

Patent Literatures

PTL 1: JP-A-2010-246421
PTL 2: JP-A-2007-105614
PTL 3: JP-A-2009-219989

Non-Patent Literature

NPL 1: Catalysis Today, 75 (2002), 145-155

Summary of Invention

Technical Problem

In the method disclosed in PTL 1, the non-ethanol raw material (i.e., the foreign matters, such as plastics) separated from papers by the mechanical separation and the pulping treatment in the ethanol production process, and the dewatered product of the residue generated in the distillation step of the fermentation broth are assumed to be incinerated. The energy recovery rate in the case where 100 t/d of municipal solid wastes are treated by waste-incineration power generation is approximately 5.5% as described in Comparative Example 1 shown later, but the value is not satisfactory, and a further enhancement of the energy recovery rate is demanded.

In the methods of PTL 2, PTL 3, and NPL 1, a satisfactory energy recovery rate cannot be obtained, and a further enhancement of the energy recovery rate is demanded.

Solution to Problem

The present inventors have accumulated earnest investigations in view of the circumstances described above, and have completed a novel method for effectively utilizing energy in a waste-incineration facility annexed with an ethanol production equipment that is capable of achieving a further enhancement of the energy recovery rate in such a manner that the waste-incineration facility is annexed not only with an ethanol production equipment and a steam turbine for power generation, but also with a methane fermentation equipment, a gas engine, and a solid oxide fuel cell (which is hereinafter referred to as "SOFC"), so as to recover the energy, and the waste heat generated therefrom is utilized as heat sources in the process steps in the ethanol production equipment.

In particular, the present invention is concerned with a method in accordance with claim 1 and a method in accordance with claim 4. Preferred embodiments are defined in the dependent claims.

The first invention is a method as defined in claim 1 for effectively utilizing energy in a waste-incineration facility annexed with an ethanol production equipment that produces bioethanol from carbohydrates contained in biomass of municipal solid wastes, containing: an ethanol production step of mixing a part of heavy components containing garbage and wet papers with pulp separated from light components containing dry papers and plastics, all of which are obtained through a pretreatment of the municipal solid wastes, and fermenting the mixture; and a methane production step of fermenting a balance of the heavy components and/or a distillation residue generated in the ethanol production step.

As one example of the municipal solid waste compositions, the national average value (which is in terms of wet weight percentage provisionally calculated from the Heisei 23rd (2011th) fiscal year data in the website of Ministry of the Environment) is 14.9% of kitchen wastes, 48.3% of papers, 9.5% of vegetation materials such as woods, none of cloths and leathers (cloths are included in papers, and leathers are included in plastics), 20.4% of plastics, and 7.0% of incombustible materials. On the other hand, the composition of a certain city (which is in terms of wet weight percentage provisionally calculated from the Heisei 24th (2012th) fiscal year data from the business summary of the environmental policy bureau of the city) is 30.4% of kitchen wastes, 36.6% of papers, 5.5% of vegetation materials such as woods, 6.7% of cloths and leathers, 16.2% of plastics, and 4.6% of incombustible materials. There is a variation between them, which can be treated by the pretreatment. Specifically, a coarse shredded matter coarsely shredded with a biaxial shredder is fed through a conveyer belt to a shredding sorter constituted by blades and a screen, and (fully) shredded with the blades rotating at a high speed, from which the plastics and the dry papers having a small specific gravity are recovered as light components with the force of wind caused by rotation, and the kitchen wastes and the wet papers having a size of the screen diameter or less and having a large specific gravity are recovered as heavy components. From the former (i.e., the light components), only pulp is further separated and recovered by the pulping treatment, and thereby the biomass capable of being used as a raw material of ethanol and biogas can be efficiently separated without the influence of the "variation in composition" and the "ratio of the heavy components and the light components". In the following description, the fraction of the heavy components that is subjected to the ethanol production is referred to as a "part of the heavy components", and the other fraction thereof is referred to as a "balance of the heavy components".

The kitchen wastes, which constitute the most of the heavy components, contain carbohydrates, such as starch, lipids, proteins, and the like, and hydrolysis of the proteins with an enzyme (protease) provides amino acids as the essential nutritional factor of yeast, which cannot be used directly as a raw material of ethanol, but promote the propagation of yeast, resulting in decrease of the amount of the yeast added as compared to the case where only pulp is used as the raw material, which leads to reduction in cost.

For the mixing ratio of the "part of the heavy components" with respect to the pulp, derived by evaluating the minimum amount of the heavy components added that is required for promoting the propagation of the yeast, the lower limit thereof is preferably 0.02, more preferably 0.05, and further preferably 0.10, and the upper limit thereof is preferably 0.50, more preferably 0.35, and further preferably 0.20, in terms of dry weight ratio with respect to the pulp assumed to be 1. Ethanol is produced from the mixture, but the mixing ratio is not limited to these ranges in all the cases.

In the invention, the biomass capable of constituting the raw material can be efficiently separated without the influence of the "variation in composition" and the "ratio of the heavy components and the light components" on the municipal solid wastes by the pretreatment, but such possibilities cannot be avoided that the municipal solid wastes contain an extremely large amount of the heavy components but the amount of the light components is small, or the municipal solid wastes are constituted only by the heavy components (particularly in foreign countries). For example, in the case where the heavy components (i.e., the garbage) contain a large amount of starch, specifically noodles, rice, breads, and the like, in addition to the wet papers, the heavy components are subjected to the ethanol production in the same manner except that amylase is added in addition to cellulase, and in the case where the heavy components contain a small amount of wet papers and starch, but is constituted mostly by lipids and proteins, the entire amount of the heavy components can be used as a raw material of the methane production. Due to the possibilities that municipal solid wastes having these compositions are used as the raw material, the ratio of the "part of the heavy components" subjected to the ethanol production and the "balance of the heavy components" subjected to the methane production is not important, and may not be determined to a particularly clear specific range, and thus it is one of the features that the "balance of the heavy components", which cannot be used as the raw material of ethanol, can be used as the raw material of the methane fermentation.

In the method for effectively utilizing energy in a waste-incineration facility annexed with an ethanol production equipment according to the first invention, waste heat is obtained by dewatering and then incinerating at least one of residues among a residue formed in the ethanol production equipment and a residue formed in the methane fermentation step is used for heat sterilization of biomass and a tank in the ethanol production equipment.

In the method for effectively utilizing energy in a waste-incineration facility annexed with an ethanol production equipment according to the first invention, it is preferred that waste heat obtained by dewatering and then incinerating at least one of residues among a residue formed in the ethanol production equipment and a residue formed in the methane fermentation step is used as a heat source of at least one step among a culturing step, a saccharifying and fermenting step, and a distilling step in the ethanol production equipment.

In the method for effectively utilizing energy in a waste-incineration facility annexed with an ethanol production equipment according to the first invention, it is preferred that steam is formed by utilizing waste heat obtained by dewatering and then incinerating at least one of residues among a residue formed in the ethanol production equipment and a residue formed in the methane fermentation step, and electric power is recovered from the steam with a steam turbine.

The waste-incineration facility in the first invention may be not only an incineration furnace of a stoker type, a fluidized bed type, a rotary kiln type, or the like, but also an conventional incineration facility of another type (associated with electric power generation), such as a gasification furnace, a melting furnace, and a gasification melting furnace combined with these.

The second invention is the method as defined in claim 4 for effectively utilizing energy in a waste-incineration facility annexed with an ethanol production equipment according to the preamble of claim 1, wherein the method further contains an energy recovery step of using the methane as a driving source of a gas engine.

In the method for effectively utilizing energy in a waste-incineration facility annexed with an ethanol production equipment according to the second invention, it is preferred that engine waste heat discharged from the gas engine and/or waste heat obtained by dewatering and then incinerating at least one of residues among a residue formed in the ethanol production equipment and a residue formed in the methane fermentation step is used for heat sterilization of biomass and/or a fermentation tank in the ethanol production equipment.

In the method for effectively utilizing energy in a waste-incineration facility annexed with an ethanol production equipment according to the second invention, it is preferred that engine waste heat discharged from the gas engine and/or waste heat obtained by dewatering and then incinerating at least one of residues among a residue formed in the ethanol production equipment and a residue formed in the methane fermentation step is used as a heat source of at least one step among a culturing step, a saccharifying and fermenting step, and a distilling step in the ethanol production equipment.

In the method for effectively utilizing energy in a waste-incineration facility annexed with an ethanol production equipment according to the second invention, it is preferred that steam is formed by utilizing engine waste heat discharged from the gas engine and/or waste heat obtained by dewatering and then incinerating at least one of residues among a residue formed in the ethanol production equipment and a residue formed in the methane fermentation step, and electric power is recovered from the steam with a steam turbine.

There is also disclosed a method for effectively utilizing energy in a waste-incineration facility annexed with an ethanol production equipment according to any one of the first and second inventions and preferred embodiments thereof, wherein the method further contains a reforming step of reforming hydrous ethanol steam generated in the ethanol production equipment, so as to form hydrogen gas; and a step of providing electric power with a fuel cell using the hydrogen gas as a hydrogen source. The hydrous ethanol preferably has an ethanol concentration of from 20 to 40% by weight, and more preferably from 25 to 35% by weight.

There is also disclosed a method for effectively utilizing energy in a waste-incineration facility annexed with an ethanol production equipment according to any one of the first and second inventions and preferred embodiments thereof, wherein the method further contains a reforming step of reforming hydrous ethanol steam generated in the ethanol production equipment and methane obtained through the methane fermenting step separately or in a mixed state, so as to form hydrogen gas; and a step of providing electric power with a fuel cell using the hydrogen gas. It is preferred that the entire amount of the hydrous ethanol steam generated in the ethanol production equipment and/or the entire amount of the methane obtained through the methane fermenting step are preferably reformed. In the fourth invention, the hydrous ethanol preferably has an ethanol concentration of from 10 to 25% by weight, and more preferably from 15 to 20% by weight.

In the above disclosed methods, the fuel cell may be a solid oxide fuel cell (SOFC), and also may be a polymer electrolyte fuel cell (PEFC), a phosphoric acid fuel cell (PAFC), a molten carbonate fuel cell (MCFC), or the like, and the hydrogen gas obtained in the reforming step may be applied to all types of fuel cells. The operating temperatures and the power generation efficiencies of the fuel cells are as shown in the following table, and the basis why the method of the invention is suitable for SOFC includes the two points, i.e., waste heat at a high temperature can be obtained (utilization in the ethanol and methane production processes), and not only the hydrogen gas obtained by the reformation but also carbon monoxide gas can be utilized (carbon monoxide gas is an inhibitory factor in fuel cells other than SOFC). SOFC has a high power generation efficiency of from 50 to 70%, but this is merely a resultant but not a basis.

Table a

| Comparison of four types of fuel cells | | | | |
| --- | --- | --- | --- | --- |
| | PEFC Polymer electrolyte type | PAFC Phosphoric acid type | MCFC Molten carbonate type | SOFC Solid oxide type |
| Operation temperature (°C) | 80-100 | 190-200 | 600-700 | 700-1,000 |
| Power generation efficiency (%) | 30-40 | 40-45 | 50-65 | 50-70 |

Advantageous Effects of Invention

According to the first invention, in a waste-incineration facility annexed with an ethanol production equipment, a part of the heavy components containing garbage and wet papers obtained by the pretreatment of municipal solid wastes and/or the distillation residue generated in the ethanol production step can be subjected to methane fermentation to form methane.

The residue obtained by removing ethanol by distillation from the broth contains lipids and proteins derived from garbage, which cannot be used directly as a raw material of ethanol, and also contains pulp that has not been enzymatically saccharified (i.e., carbohydrates), dead bodies of the yeast, or the like, and these materials are capable of constituting a methane fermentation substrate. Accordingly, the distillation residue and a part of the heavy components containing garbage and wet papers that are not subjected to the ethanol fermentation are subjected to the methane fermentation jointly, thereby achieving recovery of energy.

In the first invention, the waste heat derived from the residue formed in the ethanol production equipment and/or the residue formed in the methane fermentation step may be used for heat sterilization of biomass and a tank in the ethanol production equipment, thereby achieving further recovery of energy.

In the first invention, the waste heat derived from the residue formed in the ethanol production equipment and/or the residue formed in the methane fermentation step may be used as a heat source of at least one step among a culturing step, a saccharifying and fermenting step, and a distilling step in the ethanol production equipment, thereby achieving further recovery of energy.

In the first invention, steam may be formed by utilizing the waste heat derived from the residue formed in the ethanol production equipment and/or the residue formed in the methane fermentation step, and electric power may be recovered from the steam with a steam turbine, thereby achieving further recovery of energy.

According to the second invention, the methane is used as a driving source of a gas engine, thereby achieving further recovery of energy (the power generation efficiency of the gas engine power generation is from 30 to 40%, and the waste heat is from 30 to 40%).

In the second invention, engine waste heat discharged from the gas engine and/or waste heat derived from the residue formed in the ethanol production equipment and/or the residue formed in the methane fermentation step may be used for heat sterilization of biomass and a tank in the ethanol production equipment, thereby achieving the enhancement of the energy recovery rate.

In the second invention, engine waste heat discharged from the gas engine and/or waste heat derived from the residue formed in the ethanol production equipment and/or the residue formed in the methane fermentation step may be used as a heat source of at least one step among a culturing step, a saccharifying and fermenting step, and a distilling step in the ethanol production equipment, thereby achieving further recovery of energy.

In the second invention, steam may be formed by utilizing engine waste heat discharged from the gas engine and/or waste heat derived from the residue formed in the ethanol production equipment and/or the residue formed in the methane fermentation step, and electric power may be recovered from the steam with a steam turbine, thereby achieving further recovery of energy.

Hydrous ethanol steam generated in the ethanol production equipment may be reformed, so as to form hydrogen gas, and electric power is provided with a fuel cell using the hydrogen gas, thereby achieving further recovery of energy.

Hydrous ethanol steam generated in the ethanol production equipment and methane obtained through the methane fermenting step may be reformed, so as to form hydrogen gas, and electric power is provided with a fuel cell using the hydrogen gas, thereby achieving further recovery of energy.

According to the inventions as described above, a significant enhancement of the energy recovery rate can be achieved.

Brief Description of Drawings

Fig. 1 is a flowsheet showing Example 1 of the invention.
Fig. 2 is a flowsheet showing Example 2 of the invention.
Fig. 3 is a flowsheet showing Example 3 of the invention.
Fig. 4 is a flowsheet showing Comparative Example 1 as an conventional technique.

Description of Embodiment

Examples specifically describing the invention and a comparative example showing an conventional technique will be shown below.

Comparative Example 1 (only waste-incineration power generation)

In Fig. 4, municipal solid wastes discharged and recovered in a state where various kinds of wastes are mixed (100 t/d, 9,915 kJ/kg, 275 MWh) are placed in a waste-incineration facility and incinerated. Waste heat generated in the waste-incineration facility is converted to steam (359 t/d, 2,991 kJ/kg, 298 MWh) with a boiler, and a part of the steam is used as process steam, whereas the balance thereof is subjected to power generation with a steam turbine. A part (18 MWh) of the electric power (33.2 MWh) is used as captive power consumption, whereas the balance (15.2 MWh) thereof is subjected to electric power selling.

The provisional calculation of the energy recovery rate calculated from the heat input of 275 MWh based on the heat amount of 9,915 kJ/kg by the incineration of the municipal solid wastes (100 t/d) as the denominator and the heat output of 15.2 MWh obtained by subtracting the captive power consumption of 18 MWh from the power generation amount of 33.2 MWh obtained by subjecting the municipal solid waste to waste-incineration power generation as the numerator is as follows.

$$(33.2-18)/275 \times 100 = 5.5 \ (\%)$$

In the provisional calculating, the compositional ratio of the municipal solid wastes of the certain city described above (in terms of wet weight percentage, 30.4% of kitchen wastes, 36.6% of papers, 5.5% of vegetation materials such as woods, 6.7% of cloths and leathers, 16.2% of plastics, and 4.6% of incombustible materials) is applied, and the energy recovery rates in Examples 1 to 3 shown below are also the provisional calculation results based on the ratio.

Example 1

In Fig. 1, municipal solid wastes discharged and recovered in a state where various kinds of wastes are mixed (100 t/d, 9,915 kJ/kg, 275 MWh) are coarsely shredded in a pretreatment step, and the resulting coarse shredded matter is separated into light components having a small specific gravity (mainly containing papers and plastics, 66.8 t/d, 13,753 kJ/kg, 255.2 MWh) and heavy components containing a large amount of water and having a large specific gravity (mainly containing kitchen wastes, i.e., garbage, 33.2 t/d) by a shredding sorter.

The heavy components are divided into two parts, and a raw material slurry (containing 16% by weight of pulp and 5% by weight of the heavy components) is prepared from one part of the heavy components (22.2 t/d, 2,192 kJ/kg, 13.5 MWh) and paper waste pulp described later, and charged in the saccharifying and fermenting tank of the ethanol production equipment.

In the ethanol production equipment, yeast is preliminarily cultured and/or mainly cultured in advance, the yeast is added to the raw material slurry along with an enzyme (cellulase), thereby hydrolyzing cellulose to monosaccharides as the constituent sugars and fermenting the monosaccharides to form a broth having an ethanol concentration of 5% by volume. The broth (the lower limit of the ethanol concentration in profitability is 4.3% by weight) obtained through the saccharifying and fermenting reaction is concentrated by distillation to approximately 90% by weight, and then further concentrated to anhydrous ethanol of 99.5% by weight or more with a dewatering membrane or the like.

The light components are subjected to a pulping treatment to defiber paper into fibers, from which plastics are removed, and the resulting paper waste pulp (109.2 t/d, 3,220 kJ/kg, 97.7 MWh) is used as a raw material of ethanol production along with the heavy components.

As described above, in the ethanol production equipment, the residue obtained by removing ethanol by distillation from the broth contains lipids and proteins derived from garbage, which cannot be used directly as a raw material of ethanol, and also contains pulp that has not been enzymatically saccharified (i.e., carbohydrates), dead bodies of the yeast, and the like, and these materials are capable of constituting

a methane fermentation substrate. Accordingly, the distillation residue (124.7 t/d, 2,246 kJ/kg, 77.8 MWh) and the balance of the heavy components (mainly containing kitchen wastes, i.e., garbage) (i.e., the part that is not subjected to the ethanol production, 11.0 t/d, 2,192 kJ/kg, 6.7 MWh) are utilized as a methane fermentation resource by subjecting to methane fermentation.

Furthermore, a gas engine is annexed to the methane fermentation equipment, and methane (11 km$^3$/d, 19,705 kJ/kg, 60.2 MWh) in the biogas obtained in the methane fermentation equipment is used as a driving source of the gas engine. According to the configuration, further recovery of energy can be achieved (the gas engine power generation of 24 MWh, the power generation efficiency of from 30 to 40%, and the waste heat of 6 MWh, from 30 to 40%).

In the waste-incineration facility, the residue (24.6 t/d, 23,052 kJ/kg, 157.5 MWh, water content of 10%) discharged from the pulping step in the ethanol production equipment and the residue (54.5 t/d, 1,116 kJ/kg, 16.9 MWh, water content of 83%) discharged from the methane fermentation step are placed generally after subjecting to a dewatering treatment, and in the case where incineration is difficult to perform only with the residues, the municipal solid wastes are directly placed therein, followed by performing incineration of the wastes.

The waste-incineration facility is annexed with a boiler using waste heat discharged from the facility and a steam turbine using steam generated in the boiler, the waste heat generated in the waste-incineration facility is converted to steam (224.2 t/d, 2,991 kJ/kg, 186.3 MWh) with the boiler, and a part of the steam is used as process steam, whereas the balance thereof is used for power generation (18.7 MWh) with the steam turbine. The boiler steam discharged from the boiler and/or the extracted steam discharged from the steam turbine are utilized as a heat source for the yeast cultivation (at approximately 30°C), the saccharification and fermentation (at approximately 40°C), and the distillation (at approximately 80°C) in the ethanol production equipment.

Example 2

In Fig. 2, hydrous ethanol steam having a concentration of 17% by weight obtained in the distillation step before concentrating to anhydrous ethanol in the ethanol production equipment and methane obtained by purifying the biogas obtained in the methane fermentation step (both in total amount) are fed to a reformer and reformed in a mixed state to generate hydrogen gas, and electric power is obtained with SOFC by using the resulting hydrogen gas. At this time, the waste heat of SOFC is 18.7 MWh, and the power generation amount thereof is 46.8 MWh.

In Example 2, the step of concentrating to anhydrous ethanol in the ethanol production equipment and the biogas power generation using methane obtained in the methane fermentation step are not performed.

The other configurations in Example 2 are the same as in Example 1.

Example 3

In Fig. 3, a half of the municipal solid wastes are fed to the ethanol production equipment, whereas the remaining half thereof are directly placed in the waste-incineration equipment of the waste-incineration facility and subjected to incineration.

Hydrous ethanol steam having a concentration of 30% by weight obtained in the distillation step before concentrating to anhydrous ethanol in the ethanol production equipment is fed to a reformer and reformed to generate hydrogen gas, and electric power is obtained with SOFC by using the resulting hydrogen gas. The power generation amount of SOFC is 8.6 MWh, the power generation amount of the steam turbine annexed to the waste-incineration equipment is 32.9 MWh, and the power generation amount of the gas engine is 12 MWh.

In Example 3, the step of concentrating to anhydrous ethanol in the ethanol production equipment is not performed.

The other configurations in Example 3 are the same as in Example 1.

The effects of the invention will be specifically described based on Examples and Comparative Example.

(1) Effect of Introduction of Methane Fermentation Step

In the method of PTL 1, for an incineration furnace having a scale of 100 t/d, 50 t/d of wastes are directly incinerated, ethanol is produced by separating a raw material of ethanol from the remaining 50 t/d thereof, and plastics, which are not a raw material, and the distillation residue are incinerated. In Example 1, ethanol and methane are produced by separating a raw material of ethanol fermentation and a raw material of methane fermentation from 100 t/d of municipal solid wastes. Plastics, which are not a raw material, and the residue generated in the methane fermentation are incinerated. Accordingly, the absolute amount of the raw material of ethanol production is increased, and the production amount of ethanol is also increased.

In Example 1, in the total amount of the heavy components (33.2 t/d), 22.2 t/d is used as the raw material of ethanol production, whereas 11 t/d is used as a raw material of methane fermentation, and thereby the energy recovery rate is increased as compared to the case where the entire amount thereof is incinerated to perform waste-incineration power generation.

The energy recovery rates in Comparative Example and Examples calculated from the heat output as the numerator and the heat input as the denominator are as follows.

In Comparative Example 1 (only waste-incineration power generation) shown in Fig. 4, only municipal solid wastes (100 t/d, 9,915 kJ/kg, 275 MWh) are placed in the waste-incineration facility. The waste heat generated in the waste-incineration facility is converted to steam (359 t/d, 2,991 kJ/kg, 298 MWh) with a boiler, and a part of the steam is used as process steam, whereas power generation (33.2 MWh) is performed with the balance thereof with a steam turbine. A part (18 MWh) of the electric power is used as captive power consumption, whereas the balance (15.2 MWh) thereof is subjected to electric power selling.

In Comparative Example 1, the provisional calculation of the energy recovery rate calculated from the heat input of 275 MWh based on the heat amount of 9,915 kJ/kg of the municipal solid wastes (100 t/d) as the denominator and the heat output of 15.2 MWh obtained by subtracting the electric power consumption of 18 MWh from the power generation amount of 33.2 MWh obtained by the waste-incineration power generation as the numerator is as follows.

$$(33.2-18)/275 \times 100 = 5.5 \ (\%)$$

In Example 1 (the ethanol production equipment and the methane fermentation equipment are annexed to the waste-incineration facility of Comparative Example 1) shown in Fig. 1, the provisional calculation of the energy recovery rate calculated from the heat input of 275 MWh based on the municipal solid wastes as the denominator and the heat output of 51.4 MWh obtained by subtracting the electric power consumption of 24.7 MWh from the sum of the heat amount of 33.4 MWh of the (anhydrous) ethanol produced, the power generation amount of 18.7 MWh with the steam turbine annexed to the waste-incineration equipment, and the power generation amount of 24 MWh with the gas engine as the numerator is as follows.

$$(33.4+24+18.7-24.7)/275 \times 100 = 18.7 \ (\%)$$

It is assumed that ethanol is not converted to electric power but is used directly as a fuel or a chemical product.

In Example 2 shown in Fig. 2, based on the same calculation as in Comparative Example 1 and Example 1, in the case where the hydrous ethanol steam obtained in the distillation step before concentrating to anhydrous ethanol in the ethanol production equipment and methane obtained in the methane fermentation step (both in total amount) are reformed separately or in a mixed state, so as to generate hydrogen gas, and the hydrogen gas is converted to electric power with SOFC, the provisional calculation of the energy recovery rate calculated from the heat input of 275 MWh by the municipal solid wastes as the denominator and the heat output of 40.8 MWh obtained by subtracting the electric power consumption of 24.7 MWh from the sum of the electric power of 46.8 MWh with SOFC and the power generation amount of 18.7 MWh with the steam turbine annexed to the waste-incineration equipment as the numerator is as follows.

$$(46.8+18.7-24.7)/275 \times 100 = 14.8 \ (\%)$$

The energy recovery rate is smaller than that of Example 1 (18.7%), and the reason therefore is that the heat amount of 33.4 MWh of ethanol is converted to electric power at a power generation efficiency of 50%. However, the

8

resulting power generation amount becomes maximum as described later.

In Example 3 shown in Fig. 3, in the case where the hydrous ethanol steam obtained in the distillation step before concentrating to anhydrous ethanol in the ethanol production equipment is reformed to generate hydrogen gas, and the hydrogen gas is converted to electric power with SOFC, the provisional calculation of the energy recovery rate calculated from the heat input of 275 MWh by the municipal solid wastes as the denominator and the heat output of 29.5 MWh obtained by subtracting the electric power consumption of 24.0 MWh from the sum of the electric power of 8.6 MWh with SOFC, the power generation amount of 32.9 MWh with the steam turbine annexed to the waste-incineration equipment, and the power generation amount of 12 MWh of the gas engine as the numerator is as follows.

$$(8.6+12+32.9-24.0)/275 \times 100 = 10.7 \ (\%)$$

As described above, the provisionally calculated energy recovery rates obtained in Examples 1 to 3 exhibit significant enhancement as compared to that obtained in Comparative Example 1.

(2) Reduction Effect of $CO_2$ Emission

In the case where only incineration of 100 t/d of municipal solid wastes is performed (which is hereinafter referred to as "incineration only"), provisionally calculated values of $CO_2$ emission in Comparative Example 1, in which the waste-incineration power generation step is added to the "incineration only", and Example 1, in which the ethanol production step, the methane fermentation step, and the gas engine power generation step are added to Comparative Example 1, are shown in Table 1-1. As compared to the "incineration only", the $CO_2$ emission in Example 1 is 140.277 kg-$CO_2$/t per the municipal solid wastes, which results in a reduction of 93.795 kg-$CO_2$/t per the municipal solid wastes from Comparative Example 1.

The provisionally calculated results of the reduction rate of $CO_2$ emission in the case where the $CO_2$ emission of the "incineration only" (comparison) is assumed to be the "$CO_2$ emission that is capable of being avoided" are shown in Table 1-2. In Example 1, the reduction rate of the $CO_2$ emission is 84% in both the incineration power generation utilizing the municipal solid wastes and the ethanol production.

As described above, the method of the invention contributes to the reduction of $CO_2$ emission.

(3) Effect of Introduction of SOFC

Embodiments of the invention include a case where the hydrous ethanol steam generated in the ethanol production equipment is reformed to form hydrogen gas, and electric power is provided with SOFC by using the hydrogen gas, and a case where the hydrous ethanol steam generated in the ethanol production equipment and methane obtained in the methane fermentation step are reformed to form hydrogen gas, and electric power is provided with SOFC by using the hydrogen gas, and the effect of introduction of SOFC shown below is obtained in both the cases.

(i) Maximization of Power Generation Amount

In the case where ethanol is used as a vehicle fuel or a raw material of chemical products, it is a general procedure that the broth obtained through the saccharifying and fermenting reaction (the lower limit of the ethanol concentration in profitability is 4.3% by weight) is concentrated by distillation to approximately 90% by weight, and then further concentrated to anhydrous ethanol of 99.5% by weight or more with a dewatering membrane or the like.

On the other hand, in the case where the power generation amount is to be maximized without applying ethanol to the aforementioned purpose, the entire amount of the non-concentrated ethanol can be applied to a hydrogen source of SOFC power generation, which has a high power generation efficiency of 50%. In this case, the hydrous ethanol steam generated in the distillation step in the ethanol production equipment is fed to a reformer, and reformed to a hydrogencontaining gas. In this case, a large amount of water is required for the reformation. In the case where methane obtained through purification of the entire amount of the biogas obtained through the methane fermentation step is also used as the hydrogen source, hydrous ethanol steam having an ethanol concentration of 17% by weight (a water concentration of 83% by weight) is required, and in the case where only ethanol is used, hydrous ethanol steam having an ethanol concentration of 30% by weight (a water concentration of 70% by weight) is required.

In Example 2 (Fig. 2), in the case where the ethanol (4.5 t/d, 33.4 MWh) produced in Example 1 (Fig. 1) is used as hydrous ethanol steam having a concentration of 17% by weight in the course of distillation, the biogas (11 km$^3$/d, 60.2 MWh) in Example 1 (Fig. 1) is used as purified methane, and the entire amounts of both of them are applied to SOFC power generation, the maximum power generation amount of 46.8 MWh is obtained as shown in the following provisional calculation expression, and thus the maximization of the power generation amount is achieved.

```
(60.2+33.4) × 0.5 (power generation efficiency) = 46.8 (MWh)
```

(ii) Cost Reduction in Ethanol Purification and Biogas Power Generation

It is not necessary to provide anhydrous ethanol of 99.5% by weight or more, which is required for the case where ethanol is used as a vehicle fuel or a raw material of chemical products, and hydrous ethanol steam in the course of distillation can be applied, thereby reducing the cost for energy required for cooling and condensation after the distillation. For example, in the case where ethanol is used as a vehicle fuel or a raw material of chemical products, it is necessary to remove impurities to the standard value, and the domestic regulatory values of various components shown in Table 2 are established as the fuel grade ethanol standard JIS K2190 (it was confirmed that the product ethanol satisfied the fuel grade ethanol standard as shown in Table 2 by such a proof experiment that a part of ethanol that was actually produced from municipal solid wastes was made anhydrous, and analyzed for the components).

In consideration of the possibility of the application to a raw material for chemical products, the ethanol distilled liquid (having an ethanol concentration of approximately 50% by weight) obtained by the proof experiment was subjected to qualitative analysis for the selected organic components having boiling points around that of ethanol shown in Table 3, and the components that indicated the possible presence thereof were subjected to quantitative analysis. As a result, as shown in Table 4, it was confirmed that impurities including methanol, 1-propanol, 1-pentanol, and the like were mixed therein in a concentration of 1,000 mg/L or more as fermentation by-products other than ethanol formed through the fermentation. These impurities can be utilized as a hydrogen source of SOFC, and in the distillation, the removal of the impurities may be less carefully performed than the case where a fuel or a raw material for chemical products is to be produced.

In the case where the entire amount of methane obtained in the methane fermentation equipment is applied to SOFC as in Example 2 shown in Fig. 2, the gas engine and the related peripheral equipments become unnecessary, and the equipment cost therefore can be reduced.

(4) Sanitary Treatment of Residue

In Example 1 shown in Fig. 1, what is treated in the waste-incineration equipment includes the residue discharged from the pulping step (the plastics having a water content of 10% by weight, 24.6 t/d, 23,052 kJ/kg, 157.5 MWh) and the residue discharged from the methane fermentation equipment (the dewatered cake obtained by dewatering the final digested liquid having a water content of 83% by weight, 54.5 t/d, 1,116 kJ/kg, 16.9 MWh). Although the latter residue contains a large amount of water, the mixture obtained by mixing both the residues can be incinerated, and while surely incinerating the mixture, waste heat generated therefrom can be utilized to provide electric power (18.7 MWh) through steam turbine power generation and waste heat (33.3 MWh) capable of being applied to the heat source for the ethanol production, as shown in the following provisional calculation expression.

```
((24.6×23,052)+(54.5×1,116))/(24.6+54.5) = 7,938 kJ/kg

                             (approximately 1,900 kcal/kg)
```

While the provisional calculation is also based on the compositional ratio of municipal solid wastes of the certain city described above, in the case, for example, where municipal solid wastes having a smaller amount of plastics than the average are accepted, it may be expected that the heat amount per weight of the mixture of the residue discharged from the pulping step and the residue discharged from the methane fermentation equipment is decreased to make incineration difficult. As a measure therefore, a part of the municipal solid wastes (100 t/d) in Fig. 1 are added to the residues. Specifically, according to the aforementioned provisional calculation expression, the municipal solid wastes are supplied to make the heat amount per total weight of the mixture of a part of the municipal solid wastes and the residues reaching 1,900 kcal/kg.

Table 1-1

| Provisional calculation results of $CO_2$ Emission (unit: kg-$CO_2$/t-municipal solid wastes) | | | | | |
|---|---|---|---|---|---|
| | | | Incineration only | Comparative Example 1 | Example 1 |
| Ethanol production process | | | - | - | 100 t/d |
| Methane fermentation process | | | - | - | 115 t/d |
| Methane fermentation power generation | | | - | - | 1,000 kWh |
| Incineration process | | | 50 t/d × 2 furnaces | 50 t/d × 2 furnaces | 40 t/d × 2 furnaces |
| Incineration power generation | | | - | 2,080 kW | 840 kW |
| Input | Collection | Installation of municipal solid wastes by vehicle | 2.435 | 2.435 | 2.435 |
| | Treatment | Incineration of plastics | 296.314 | 296.314 | 296.314 |
| | | Consumed electric power | 95.642 | 106.269 | 141.567 |
| | | Consumed fuel (for start up and shutdown of incineration furnace) | 5.825 | 5.825 | 3.475 |
| | | Consumed water and chemicals | 8.367 | 8.367 | 15.926 |
| Output | Disposal | Export of ash by vehicle | 0.294 | 0.294 | 0.257 |
| | | Landfill of ash | 0.367 | 0.367 | 0.321 |
| | | Discharge to sewage line | 0.057 | 0.057 | 0.213 |
| Subtotal | | | 409.301 | 419.928 | 460.509 |
| Output | Supply | Transport of ethanol by vehicle | 0.000 | 0.000 | 0.080 |
| | | Utilization of ethanol | 0.000 | 0.000 | -88.211 |
| | | Power generation amount | 0.000 | -185.856 | -232.101 |
| Total | | | 409.301 | 234.072 | 140.277 |

Note 1) The calculation is made with an assumption that the $CO_2$ emission of the vehicle carrying the municipal solid wastes is 0.487 kg-$CO_2$/km·vehicle, and the $CO_2$ emission of the vehicle carrying the ash, ethanol, or RPF is 1.38 kg-$CO_2$/km·vehicle. Note 2) Ethanol is used as a substitute of gasoline, and the $CO_2$ emission unit converted to heat amount is used.

Table 1-2

| Reduction of $CO_2$ Emission | | | |
|---|---|---|---|
| Item | Comparative Example 1 | Example 1 | Unit |
| $CO_2$ emission of processing method | 419. 928 | 460.509 | kg-$CO_2$/t |
| $CO_2$ emission avoided | 409.301 | 409.301 | kg-$CO_2$/t |
| Increment of $CO_2$ emission by use of processing method | 10.627 | 51.208 | kg-$CO_2$/t |
| Ethanol production amount | 0.000 | 57.650 | L/t |
| $CO_2$ emission per ethanol consumption rate | 0.000 | 0.244 | kg-$CO_2$/t |
| $CO_2$ emission rate as comparison | 1.530 | 1.530 | kg-$CO_2$/t |
| Reduction rate of $CO_2$ emission by ethanol production | 0.0 | 84.0 | % |
| Electric energy | 334.875 | 418.200 | kWh/t |
| $CO_2$ emission per electric power consumption rate | 0.032 | 0.089 | kg-$CO_2$/kWh |

(continued)

| Reduction of CO₂ Emission | | | |
|---|---|---|---|
| Item | Comparative Example 1 | Example 1 | Unit |
| $CO_2$ emission rate as comparison | 0.555 | 0.555 | kg-$CO_2$/kWh |
| Reduction rate of $CO_2$ emission by incineration power generation | 94.2 | 84.0 | % |

Table 2

| Standard of fuel grade ethanol | | |
|---|---|---|
| Item | JIS K2190 | Product ethanol |
| Appearance | colorless or pale yellow transparent without suspended or floating matter | colorless without suspended or floating matter |
| Alcohol content (vol%) | 99.5 or more | 99.9 |
| Methanol (g/L) | 4.0 or less | 1.2 |
| Water content (wt%) | 0.70 or less | 0.07 |
| Organic impurities (g/L) | 10 or less | < 10 |
| Electroconductivity (mS/m) | 500 or less | < 500 |
| Volatile component (mg/ 100 mL) | 5.0 or less | < 5 |
| Copper (mg/kg) | 0.10 or less | 0.06 |
| Acid degree (wt%) | 0.0070 or less | 0.0024 |
| Sulfur content (mg/kg) | 10 or less | < 10 |

Table 3

| Components applied to qualitative analysis based on results of quantitative analysis | | |
|---|---|---|
| Peak No. | Estimated component | Boiling point (°C) |
| 1 | water | 100 |
| 2 | methanol | 64 |
| 3 | ethanol | 78 |
| 4 | 2-propanol | 82.4 |
| 5 | acetone | 56 |
| 6 | 1-propanol | 97-98 |
| 7 | 2-butanol | 99 |
| 8 | ethyl acetate | 77 |
| 9 | isobutyl alcohol | 108 |
| 10 | butyl formate | 105-107 |
| 11 | 1-butanol | 117 |
| 12 | acetal | 103 |
| 13 | 1-pentanol | 138 |

(continued)

| Components applied to qualitative analysis based on results of quantitative analysis | | |
|---|---|---|
| Peak No. | Estimated component | Boiling point (°C) |
| 14 | 3-methyl-1-butanol | 131.1 |
| 15 | 2-methyl-1-butanol | 136-138 |

Table 4

| Results of quantitative analysis | | |
|---|---|---|
| Item | Unit | Value |
| water | % by weight | 35.0 |
| methanol | mg/L | 2,300 |
| ethanol *1 | g/L | 570 |
| ethanol *2 | w/v% | 60.2 |
| 2-propanol | mg/L | 99 |
| acetone | mg/L | 17 |
| 1-propanol | mg/L | 1,400 |
| 2-butanol | mg/L | 23 |
| ethyl acetate | mg/L | 200 |
| isobutyl alcohol | mg/L | 580 |
| butyl formate | mg/L | < 5 |
| 1-butanol | mg/L | 27 |
| acetal | mg/L | < 5 |
| 1-pentanol | mg/L | 1,300 |
| 3-methyl-1-butanol | mg/L | < 5 |
| 2-methyl-1-butanol | mg/L | 630 |
| *1: gas chromatography method *2: alcohol meter | | |

**Claims**

1. A method for utilizing energy in a waste-incineration facility annexed with an ethanol production equipment that forms bioethanol from carbohydrates contained in biomass of municipal solid wastes, comprising: an ethanol production step of mixing a part of heavy components containing garbage and wet papers with pulp separated from light components containing dry papers and plastics, all of which are obtained through a pretreatment of the municipal solid wastes, and fermenting the mixture; and a methane production step of fermenting a balance of the heavy components and/or a distillation residue generated in the ethanol production step, **characterized in that** waste heat obtained by dewatering and then incinerating at least one of residues among a residue formed in the ethanol production step and a residue formed in the methane production step is used for heat sterilization of biomass and a tank in the ethanol production equipment.

2. The method for utilizing energy in a waste-incineration facility annexed with an ethanol production equipment according to claim 1, wherein waste heat obtained by dewatering and then incinerating at least one of residues among a residue formed in the ethanol production step and a residue formed in the methane production step is used as a heat source of at least one step among a culturing step, a saccharifying and fermenting step, and a distilling step in the ethanol production equipment.

3. The method for utilizing energy in a waste-incineration facility annexed with an ethanol production equipment according to claim 1 or 2, wherein steam is formed by utilizing waste heat obtained by dewatering and then incinerating at least one of residues among a residue formed in the ethanol production step and a residue formed in the methane production step, and electric power is recovered from the steam with a steam turbine.

4. A method for utilizing energy in a waste-incineration facility annexed with an ethanol production equipment that forms bioethanol from carbohydrates contained in biomass of municipal solid wastes, comprising: an ethanol production step of mixing a part of heavy components containing garbage and wet papers with pulp separated from light components containing dry papers and plastics, all of which are obtained through a pretreatment of the municipal solid wastes, and fermenting the mixture; and a methane production step of fermenting a balance of the heavy components and/or a distillation residue generated in the ethanol production step,
**characterized in that** the method further comprises an energy recovery step of using the methane as a driving source of a gas engine.

5. The method for utilizing energy in a waste-incineration facility annexed with an ethanol production equipment according to claim 4, wherein engine waste heat discharged from the gas engine and/or waste heat obtained by dewatering and then incinerating at least one of residues among a residue formed in the ethanol production step and a residue formed in the methane production step is used for heat sterilization of biomass and/or a fermentation tank in the ethanol production equipment.

6. The method for utilizing energy in a waste-incineration facility annexed with an ethanol production equipment according to claim 4 or 5, wherein engine waste heat discharged from the gas engine and/or waste heat obtained by dewatering and then incinerating at least one of residues among a residue formed in the ethanol production step and a residue formed in the methane production step is used as a heat source of at least one step among a culturing step, a saccharifying and fermenting step, and a distilling step in the ethanol production equipment.

7. The method for utilizing energy in a waste-incineration facility annexed with an ethanol production equipment according to any one of claims 4 to 6, wherein steam is formed by utilizing engine waste heat discharged from the gas engine and/or waste heat obtained by dewatering and then incinerating at least one of residues among a residue formed in the ethanol production step and a residue formed in the methane production step, and electric power is recovered from the steam with a steam turbine.

8. The method for utilizing energy in a waste-incineration facility annexed with an ethanol production equipment according to any one of claims 1 to 7, wherein the method further comprises a reforming step of reforming hydrous ethanol steam in the course of distillation generated in the ethanol production step, so as to form hydrogen gas; and a step of providing electric power with a fuel cell using the hydrogen gas.

9. The method for utilizing energy in a waste-incineration facility annexed with an ethanol production equipment according to any one of claims 1 to 7, wherein the method further comprises a reforming step of reforming hydrous ethanol steam in the course of distillation generated in the ethanol production step and methane obtained through the methane production step separately or in a mixed state, so as to form hydrogen gas; and a step of providing electric power with a fuel cell using the hydrogen gas.

**Patentansprüche**

1. Verfahren zur Nutzung von Energie in einer Abfallverbrennungsanlage, die mit einer Ethanol-Produktionsvorrichtung verbunden ist, die Bioethanol aus Kohlenhydraten bildet, die in der Biomasse von kommunalen Feststoffabfällen enthalten sind, umfassend: einen Ethanol-Produktionsschritt des Mischens eines Teils von schweren Komponenten, die Müll und nasse Papiere enthalten, mit Zellstoff, der von leichten Komponenten getrennt wird, die trockene Papiere und Plastik enthalten, die alle durch eine Vorbehandlung der kommunalen Feststoffabfälle erhalten werden, und des Fermentierens der Mischung; und einen Methan-Produktionsschritt des Fermentierens eines Rests der schweren Komponenten und/oder eines Destillationsrückstands, der im Ethanol-Produktionsschritt erzeugt wird,
**dadurch gekennzeichnet, dass** Abwärme, die durch Entwässern und dann Verbrennen von mindestens einem von Rückständen unter einem im Ethanol-Produktionsschritt gebildeten Rückstand und einem im Methan-Produktionsschritt gebildeten Rückstand erhalten wird, zur Hitzesterilisation von Biomasse und einen Tank in der Ethanol-Produktionsvorrichtung verwendet wird.

2. Das Verfahren zur Nutzung von Energie in einer Abfallverbrennungsanlage, die mit einer Ethanol-Produktionsvorrichtung nach Anspruch 1 verbunden ist, wobei Abwärme, die durch Entwässern und dann Verbrennen von mindestens einem von Rückständen unter einem im Ethanol-Produktionsschritt gebildeten Rückstand und einem im Methan-Produktionsschritt gebildeten Rückstand erhalten wird, als eine Wärmequelle für mindestens einen Schritt unter einem Kultivierungsschritt, einem Verzuckerungs- und Gärungsschritt und einem Destillationsschritt in der Ethanol-Produktionsvorrichtung verwendet wird.

3. Das Verfahren zur Nutzung von Energie in einer Abfallverbrennungsanlage, die mit einer Ethanol-Produktionsvorrichtung nach Anspruch 1 oder 2 verbunden ist, wobei Dampf durch Nutzung von Abwärme gebildet wird, die durch Entwässern und dann Verbrennen von mindestens einem von Rückständen unter einem im Ethanol-Produktionsschritt gebildeten Rückstand und einem im Methan-Produktionsschritt gebildeten Rückstand erhalten wird, und elektrische Energie aus dem Dampf mit einer Dampfturbine zurückgewonnen wird.

4. Verfahren zur Nutzung von Energie in einer Abfallverbrennungsanlage, die mit einer Ethanol-Produktionsvorrichtung verbunden ist, die Bioethanol aus Kohlenhydraten bildet, die in der Biomasse von kommunalen Feststoffabfällen enthalten sind, umfassend: einen Ethanol-Produktionsschritt des Mischens eines Teils von schweren Komponenten, die Müll und nasse Papiere enthalten, mit Zellstoff, der von leichten Komponenten getrennt wird, die trockene Papiere und Plastik enthalten, die alle durch eine Vorbehandlung der kommunalen Feststoffabfälle erhalten werden, und des Fermentierens der Mischung; und einen Methan-Produktionsschritt des Fermentierens eines Rests der schweren Komponenten und/oder eines Destillationsrückstands, der im Ethanol-Produktionsschritt erzeugt wird, **dadurch gekennzeichnet, dass** das Verfahren weiter einen Energierückgewinnungsschritt des Verwendens des Methans als eine Antriebsquelle eines Gasmotors umfasst.

5. Das Verfahren zur Nutzung von Energie in einer Abfallverbrennungsanlage, die mit einer Ethanol-Produktionsvorrichtung nach Anspruch 4 verbunden ist, wobei Motorabwärme, die vom Gasmotor abgegeben wird, und/oder Abwärme, die durch Entwässern und dann Verbrennen von mindestens einem von Rückständen unter einem im Ethanol-Produktionsschritt gebildeten Rückstand und einem im Methan-Produktionsschritt gebildeten Rückstand erhalten wird, zur Hitzesterilisation von Biomasse und/oder einen Fermentationstank in der Ethanol-Produktionsvorrichtung verwendet wird.

6. Das Verfahren zur Nutzung von Energie in einer Abfallverbrennungsanlage, die mit einer Ethanol-Produktionsvorrichtung nach Anspruch 4 oder 5 verbunden ist, wobei Motorabwärme, die vom Gasmotor abgegeben wird, und/oder Abwärme, die durch Entwässern und dann Verbrennen von mindestens einem von Rückständen unter einem im Ethanol-Produktionsschritt gebildeten Rückstand und einem im Methan-Produktionsschritt gebildeten Rückstand erhalten wird, als eine Wärmequelle für mindestens einen Schritt unter einem Kultivierungsschritt, einem Verzuckerungs- und Gärungsschritt und einem Destillationsschritt in der Ethanol-Produktionsvorrichtung verwendet wird.

7. Das Verfahren zur Nutzung von Energie in einer Abfallverbrennungsanlage, die mit einer Ethanol-Produktionsvorrichtung nach irgendeinem der Ansprüche 4 bis 6 verbunden ist, wobei Dampf durch Nutzung der von dem Gasmotor abgegebenen Motorabwärme und/oder Abwärme, die durch Entwässern und dann Verbrennen von mindestens einem von Rückständen unter einem im Ethanol-Produktionsschritt gebildeten Rückstand und einem im Methan-Produktionsschritt gebildeten Rückstand gebildet wird, und elektrische Energie aus dem Dampf mit einer Dampfturbine zurückgewonnen wird.

8. Das Verfahren zur Nutzung von Energie in einer Abfallverbrennungsanlage, die mit einer Ethanol-Produktionsvorrichtung nach irgendeinem der Ansprüche 1 bis 7 verbunden ist, wobei das Verfahren weiter einen Reformierungsschritt des Reformierens von wasserhaltigem Ethanoldampf im Verlauf der Destillation, erzeugt im Ethanol-Produktionsschritt, umfasst, um Wasserstoffgas zu bilden; und einen Schritt des Bereitstellens von elektrischer Energie mit einer Brennstoffzelle unter Verwenden des Wasserstoffgases.

9. Das Verfahren zur Nutzung von Energie in einer Abfallverbrennungsanlage, die mit einer Ethanol-Produktionsvorrichtung nach irgendeinem der Ansprüche 1 bis 7 verbunden ist, wobei das Verfahren weiter einen Reformierungsschritt des Reformierens von wasserhaltigem Ethanoldampf im Verlauf der Destillation, erzeugt in Ethanol-Produktionsschritt, und von Methan, das durch den Methan-Produktionsschritt getrennt oder in einem gemischten Zustand erhalten wird, umfasst, um Wasserstoffgas zu bilden; und einen Schritt des Bereitstellens von elektrischer Energie mit einer Brennstoffzelle unter Verwenden des Wasserstoffgases.

**Revendications**

1. Procédé d'utilisation de l'énergie dans une installation d'incinération de déchets annexée à un équipement de production d'éthanol qui forme du bioéthanol à partir d'hydrates de carbone contenus dans la biomasse de déchets solides municipaux, comprenant: une étape de production d'éthanol consistant à mélanger une partie des composants lourds contenant des déchets et des papiers humides avec de la pâte à papier séparée des composants légers contenant des papiers secs et des plastiques, qui sont tous obtenus par un prétraitement des déchets solides municipaux, et à faire fermenter le mélange; et une étape de production de méthane consistant à faire fermenter un reste des composants lourds et/ou un résidu de distillation généré dans l'étape de production d'éthanol, **caractérisé en ce que** la chaleur résiduelle obtenue par déshydratation puis incinération d'au moins un des résidus parmi un résidu formé dans l'étape de production d'éthanol et un résidu formé dans l'étape de production de méthane est utilisée pour la stérilisation thermique de la biomasse et d'un réservoir dans l'équipement de production d'éthanol.

2. Procédé d'utilisation de l'énergie dans une installation d'incinération de déchets annexée à un équipement de production d'éthanol selon la revendication 1, dans lequel la chaleur résiduelle obtenue par déshydratation puis incinération d'au moins un des résidus parmi un résidu formé dans l'étape de production d'éthanol et un résidu formé dans l'étape de production de méthane est utilisée comme source de chaleur d'au moins une étape parmi une étape de culture, une étape de saccharification et de fermentation, et une étape de distillation dans l'équipement de production d'éthanol.

3. Procédé d'utilisation de l'énergie dans une installation d'incinération des déchets annexée à un équipement de production d'éthanol selon les revendications 1 ou 2, dans lequel la vapeur est formée en utilisant la chaleur résiduelle obtenue par déshydratation et ensuite incinération d'au moins un des résidus parmi un résidu formé dans l'étape de production d'éthanol et un résidu formé dans l'étape de production de méthane, et l'énergie électrique est récupérée de la vapeur avec une turbine à vapeur.

4. Procédé d'utilisation de l'énergie dans une installation d'incinération des déchets annexée à un équipement de production d'éthanol qui forme du bioéthanol à partir d'hydrates de carbone contenus dans la biomasse de déchets solides municipaux, comprenant: une étape de production d'éthanol consistant à mélanger une partie des composants lourds contenant des déchets et des papiers humides avec de la pâte à papier séparée des composants légers contenant des papiers secs et des plastiques, qui sont tous obtenus par un prétraitement des déchets solides municipaux, et à faire fermenter le mélange; et une étape de production de méthane consistant à faire fermenter le reste des composants lourds et/ou un résidu de distillation généré lors de l'étape de production d'éthanol, **caractérisé en ce que** le procédé comprend en outre une étape de récupération d'énergie consistant à utiliser le méthane comme source d'entraînement d'un moteur à gaz.

5. Procédé d'utilisation de l'énergie dans une installation d'incinération de déchets annexée à un équipement de production d'éthanol selon la revendication 4, dans lequel la chaleur résiduelle du moteur déchargée du moteur à gaz et/ou la chaleur résiduelle obtenue par déshydratation puis incinération d'au moins un des résidus parmi un résidu formé dans l'étape de production d'éthanol et un résidu formé dans l'étape de production de méthane est utilisée pour la stérilisation thermique de la biomasse et/ou d'un réservoir de fermentation dans l'équipement de production d'éthanol.

6. Procédé d'utilisation de l'énergie dans une installation d'incinération de déchets annexée à un équipement de production d'éthanol selon les revendications 4 ou 5, dans lequel la chaleur résiduelle du moteur déchargée du moteur à gaz et/ou la chaleur résiduelle obtenue par déshydratation puis incinération d'au moins un des résidus parmi un résidu formé dans l'étape de production d'éthanol et un résidu formé dans l'étape de production de méthane est utilisée comme source de chaleur d'au moins une étape parmi une étape de culture, une étape de saccharification et de fermentation, et une étape de distillation dans l'équipement de production d'éthanol.

7. Procédé d'utilisation de l'énergie dans une installation d'incinération des déchets annexée à un équipement de production d'éthanol selon l'une quelconque des revendications 4 à 6, dans lequel de la vapeur est formée en utilisant la chaleur résiduelle du moteur déchargée du moteur à gaz et/ou la chaleur résiduelle obtenue par déshydratation puis incinération d'au moins un des résidus parmi un résidu formé dans l'étape de production d'éthanol et un résidu formé dans l'étape de production de méthane, et l'énergie électrique est récupérée de la vapeur avec une turbine à vapeur.

8. Procédé d'utilisation de l'énergie dans une installation d'incinération des déchets annexée à un équipement de production d'éthanol selon l'une quelconque des revendications 1 à 7, dans lequel le procédé comprend en outre une étape de reformage consistant à reformer la vapeur d'éthanol hydratée au cours de la distillation générée dans l'étape de production d'éthanol, de manière à former de l'hydrogène gazeux; et une étape de fourniture d'énergie électrique avec une pile à combustible utilisant l'hydrogène gazeux.

9. Procédé d'utilisation de l'énergie dans une installation d'incinération de déchets annexée à un équipement de production d'éthanol selon l'une quelconque des revendications 1 à 7, dans lequel le procédé comprend en outre une étape de reformage de la vapeur d'éthanol hydratée au cours de la distillation générée dans l'étape de production d'éthanol et du méthane obtenu par l'étape de production de méthane séparément ou à l'état mélangé, de manière à former de l'hydrogène gazeux; et une étape d'alimentation en énergie électrique avec une pile à combustible utilisant l'hydrogène gazeux.

Fig.1

municipal solid wastes
100t/d
9,915kJ/kg
275MWh

waste·incineration facility

ethanol production equipment

waste-incineration equipment
waste heat

boiler
224.2t/d
2,991kJ/kg
186.3MWh

boiler steam

steam turbine

extracted steam

electric power
18.7MWh

waste heat

residue
24.6t/d
23,052kJ/kg
157.5MWh

pretreatment step

light components

heavy components
33.2t/d

pulping

paper waste pulp
109.2t/d water 65%
3,220kJ/kg
97.7MWh

culturing

saccharifying and fermenting
22.2t/d water 50%
2,192kJ/kg
13.5MWh

distillation

distillation residue
124.7t/d
2,246kJ/kg
77.8MWh

residue
54.5t/d
1,116kJ/kg
16.9MWh

methane fermentation equipment

biogas
11km³/d
19,705kJ/kg
60.2MWh

gas engine

electric power
24MWh

waste heat
6MWh

CO₂

ethanol
11.0t/d
2,192kJ/kg
6.7MWh
4.5t/d
33.4MWh

Mark ◇ means waste heat is used as a heat source for heat sterilization, distillation or the like in the ethanol production equipment.

Fig. 2

Mark ◇ has the same meaning as in Fig.1

Fig. 3

Fig. 3 — Flow diagram:

municipal solid wastes
100t/d
9,915kJ/kg
275MWh

ethanol production equipment
pretreatment step
light components
heavy components
31.4t/d
13,753kJ/kg
127.6MWh

pulping
culturing
paper waste pulp
saccharifying and fermenting
distillation

16.6t/d
11.1t/d water 50%
2,192kJ/kg — 6.8MWh
CO₂

31.7t/d water 65%
5553kJ/kg
48.9MWh

62.6t/d
2,246kJ/kg
39.1MWh
distillation residue

residue
12.3t/d
23,052kJ/kg
78.7MWh

50t/d
9,915kJ/kg
138MWh

waste-incineration facility
waste-incineration equipment
waste heat
boiler
298.6t/d
2,991kJ/kg
248.1MWh
boiler steam
steam turbine
extracted steam
electric power
32.9MWh
waste heat

methane fermentation equipment
residue
27.2t/d
1,116kJ/kg
8.5MWh
gas engine
3MWh
electric power
12MWh
biogas
5.5km³/d/d
19,705kJ/kg
30.1MWh

hydrous ethanol steam
5.5t/d
2,192kJ/kg
3.4MWh
reformer
hydrogen
SOFC equipment
electric power
8.6MWh

Mark ◇ has the same meaning as in Fig.1

Fig.4

```
                                    ┌─────────────────────────┐   100t/d
                                    │  municipal solid wastes │   9,915kJ/kg
                                    └─────────────────────────┘   275MWh
                                                 ┊
  waste-incineration facility                    ┊
                                    ╭────────────▼────────────╮
                                    │ ┌─────────────────────┐ │
                                    │ │waste-incineration equipment│ │
                                    │ └─────────────────────┘ │
                                    ╰──────────────┊──────────╯
                                           ┊  waste heat
                              waste heat ▼ ┊
                                    ┌──────▼──────┐
                                    │   boiler    │
                                    └──────┬──────┘        359t/d
                                           │               2,991kJ/kg
  process steam ◄──────────────────┌──────▼──────┐         298MWh
                                    │ boiler steam│
                                    └──────┬──────┘
                                    ┌──────▼──────┐
                                    │steam turbine│
                                    └──────┬──────┘
                                    ┌──────▼──────┐
                                    │electric power│
                                    └─────────────┘
                                       33.2MWh
```

**EP 3 335 809 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007005954 A1
- JP 2005211805 A
- JP 2010246421 A
- JP 2007105614 A
- JP 2009219989 A

**Non-patent literature cited in the description**

- *Catalysis Today,* 2002, vol. 75, 145-155